# EUROPÄISCHE PATENTANMELDUNG

(11) **EP 3 069 661 A1**
(43) Veröffentlichungstag der Anmeldung: **21.09.2016**
(21) Anmeldenummer: 15020038.4
(22) Anmeldetag: 17.03.2015
(51) Int. Cl.: A61B 17/00, A61B 34/10

(54) **PATIENTENSPEZIFISCH GEFORMTER OKKLUDER**

(71) Anmelder: Peter Osypka Stiftung, 79639 Grenzach-Wyhlen (DE)
(72) Erfinder: Osypka, Peter, 79639 Grenzach-Wyhlen (DE)

(57) **Zusammenfassung**

Die Erfindung betrifft einen Patienten spezifisch geformten Okkluder geeignet zum Verschließen von ungewollten Öffnungen im Herzen, wobei der Okkluder von einer kompakten Erscheinungsform in eine expandierte Erscheinungsform überführbar ist, wobei der Okkluder eine erste Verschlussscheibe (1) und eine zweite Verschlussscheibe (2) aufweist, welche in der expandierten Erscheinungsform auf je einer Seite der Öffnung anliegen, und wobei der Okkluder ferner einen Kopplungsteil (3) aufweist welcher die beiden Verschlussscheiben verbindet. D dadurch gekennzeichnet dass die Außenkontur und Größe mindestens einer der Verschlussscheiben der Außenkontur und Größe des Septum-Defekts (10) entspricht, wobei ein Patienten spezifisch erstelltes Modell des Septum-Defekts zur Herstellung und Formgebung der Verschlussscheiben dient.

## Beschreibung

Die Erfindung betrifft einen Patienten spezifisch geformten Okkluder zum Verschließen von ungewollten Öffnungen im Herzen.

Verschlüsse für ungewollte Öffnungen im Herzen, sogenannte Okkluder sind bekannt. Zahlreiche Patentveröffentlichungen betreffen Okkluder. Das Europäische Patent EP2531113B1 (Osypka) beschreibt beispielsweise einen Okkluder, der zwei gegeneinander beabstandete flexible Verschlussscheiben aufweist, die aus einem Geflecht von Metalldrähten gebildet sind. Die Verbindung zwischen den Verschlussscheiben ist aufgrund einer speziellen Verschweißung frei von Klammern, Fassungen oder Gewindehülsen.

Okkluder werden beispielsweise zum Verschließen eines Atriumseptumdefekts (ASD) oder eines Defekts im Foramen ovale (PFO) eingesetzt. Okkluder können auch bei Ventrikelseptumdefekten (VSD) eingesetzt werden.

Der Atriumseptumdefekt (ASD) oder Vorhofseptumdefekt ist ein Loch in der Herzscheidewand zwischen den beiden Vorhöfen des Herzens. Der ASD ist mit ca. 7,5% aller angeborenen Herzfehler die dritthäufigste angeborene Herzfehlbildung.

Die häufigste Form des Vorhofseptumdefekts ist der sogenannte ASD II. Das Loch (Shunt) liegt in der Mitte der Vorhofscheidewand und je nach Größe fließt wegen des Druckunterschiedes in den Herzkammern eine mehr oder weniger große Blutmenge vom linken in den rechten Vorhof, also wieder in den Lungenkreislauf. In den ersten Lebensjahren ist eine pulmonale Hypertonie (Lungenhochdruck) durch den Shunt nicht zu erwarten, da der Druckunterschied in den Vorhöfen nicht sehr bedeutsam ist. Im jugendlichen- oder fortgeschrittenen Erwachsenenalter treten meist Beschwerden auf. Deshalb wird das Loch in der Vorhofscheidewand heute meist durch einen Okkluder verschlossen.

Die zweit häufigste Form eines Septum-Defekts ist der Ostium-Primum-Defekt auch ASD I genannt. Das Loch liegt im unteren Teil der Vorhofscheidewand, reicht bis an die Klappenebene heran und ist oft mit einer Fehlfunktion der Mitralklappe (zwischen linkem Vorhof und linkem Ventrikel) verbunden, seltener mit einer Fehlfunktion der Trikuspidalklappe (zwischen rechtem Vorhof und rechter Herzkammer). Zugrunde liegt diesem Herzfehler eine Hemmung der Endokardkissenbildung. Der ASD I wir derzeit immer chirurgisch mit einem Patch aus körpereigenem Gewebe oder mit Kunststoff verschlossen.

Ein ASD tritt auch außer den oben beschriebenen singulären Formen im Zusammenhang mit anderen Fehlbildungen auf.

Eine sehr seltene Form des ASD ist der Sinus-Venosus Defekt. Der Defekt liegt im oberen Anteil der Vorhofscheidenwand und in ca. 90% der Fälle werden eine oder mehrere in den rechten Vorhof oder in die obere Hohlvene fehleinmündende Lungenvenen nachgewiesen. Auch dieser Defekt wird chirurgisch mit einem Patch verschlossen und die fehleinmündenden Lungenvenen werden so umgesetzt, dass der normale Blutfluss gewährleistet ist.

Das Foramen ovale ist eine türartige Verbindung zwischen dem linken und dem rechten Vorhof, die im vorgeburtlichen Kreislauf den Blutübertritt von rechts (Lungenkreislauf) nach links (Körperkreislauf) zulässt. Das Foramen ovale verschließt sich normalerweise in den ersten Lebenstagen oder Wochen. Geschieht dies nicht spricht man von einem persistierenden (anhaltenden) Foramen ovale (PFO).

Zur Behandlung von atrialen Septum-Defekten (ASD), insbesondere für den minimalinvasiven Verschluss wird der AMPLATZER™ Septal Occluder eingesetzt. Diese doppel-scheibenförmigen Okkluder bestehen aus einem Drahtgeflecht aus Nitinol mit Polyestergewebe. Sie wurden konstruiert, um sich beidseitig sicher an die Wand des Septums anzulegen, den Defekt zu verschließen und eine Plattform für das Einwachsen von Gewebe nach der Implantation zu bieten. Je nach Anatomie stehen verschiedene Occluder Modelle und Größen zur Verfügung. Nach Überwachsen mit Herzinnenhaut (Endokard) ist das Loch dauerhaft verschlossen. Voraussetzung dafür ist, dass das Loch nicht zu groß ist, mittig liegt und glatte Ränder hat. Sind diese Voraussetzungen nicht gegeben muss das Loch chirurgisch verschlossen werden. Zum Verschluss des PFO wird ebenfalls ein Amplatzer Okkluder eingesetzt.

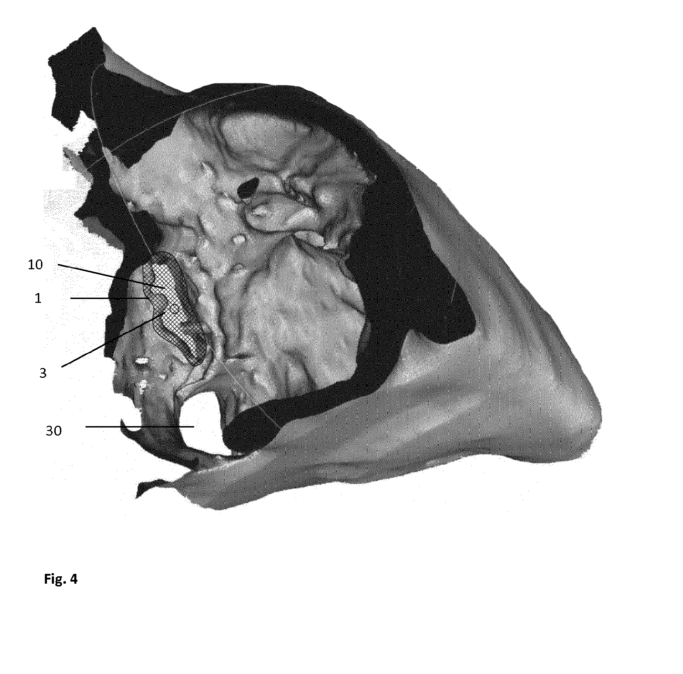

Mit modernster 3D-Echokardiographie können heute Defekte sehr sicher dargestellt und vermessen werden. Wie in Fig. 4 dargestellt, ist die Form und Größe des Defekts komplex. Um den in Fig. 4 dargestellten Defekt sicher zu verschließen ist eine relativ große Verschlussscheibe erforderlich.

Die amerikanische Zulassungsbehörde FDA berichtet im Magazin DAIC (Diagnostic and interventional Cardiology) von Erosionen des Herzgewebes beim Einsatz übergroßer Okkluder. (13. Oktober 2013). Die Erosion ist dadurch hervorgerufen dass bei schlagendem Herzen das Okkludergeflecht an der Herzwand oder der Aortawand reibt.

Es ist eine Aufgabe der Erfindung, einen verbesserten Okkluder zu entwickeln, der eine Anpassung an die anatomische Form des zu verschließenden Septum-Defekts und eine Anpassung an die Lage des Defekts im Vorhof ermöglicht. Der Okkluder soll das umliegende Gewebe des Septum-Defekts, insbesondere im Bereich der Aorta minimal bis gar nicht beeinträchtigen aber dennoch den Defekt sicher verschließen. Der Okkluder soll es ermöglichen, dass auch solche Löcher in der Vorhofscheidewand verschlossen werden können, die nicht in der Mitte liegen, keine glatten Ränder haben, zu groß sind und die wie oben beschrieben derzeit chirurgisch verschlossen werden müssen.

Ferner soll der Okkluder den minimalinvasiven Verschluss des ASD I ermöglichen.

Die Aufgabe wird gelöst durch einen Okkluder aus Formgedächtnismaterial, wobei die Verschlussscheiben des Okkluders in ihrer Form und Größe individuell an die Außenkontur und die Größe des Septum-Defekts eines jeden Patienten angepasst sind und somit der Okkluder der Anatomie des Septum-Defekts maßstabsgetreu entspricht.

Die vorliegende Erfindung betrifft einen Patienten spezifisch geformten Okkluder geeignet zum Verschließen einer ungewollten Öffnung im Herzen wobei der Okkluder von einer kompakten Erscheinungsform in eine expandierte Erscheinungsform überführbar ist, wobei der Okkluder eine erste Verschlussscheibe und eine zweite Verschlussscheibe aufweist, welche in der expandierten Erscheinungsform auf je einer Seite der Öffnung anliegen, und wobei der Okkluder ferner einen Kopplungsteil aufweist welcher die beiden Verschlussscheiben verbindet, dadurch gekennzeichnet dass die Außenkontur und Größe mindestens einer der Verschlussscheiben der Außenkontur und Größe des Septum-Defekts entspricht, wobei ein Patienten spezifisch erstelltes Modell des Septum-Defekts zur Herstellung und Formgebung der Verschlussscheiben dient.

Das Patienten spezifisch erstellte Modell des Septum-Defekts wird auf Basis von digitalen Aufnahmen des Septum-Defekts eines jeden Patienten erstellt. Der Septum-Defekt unterscheidet sich in Form und Größe von Patient zu Patient. Somit wird für jeden Patient sein ganz persönliches Modell des Septum-Defekts erstellt.

Beim Verschließen eines PFO sind zentrisch versetzte Scheiben von Vorteil.

Die exakte Größe des PFO oder des ASD wird beispielsweise durch Ultraschall, transösophagiale Echokardiografie (TEE), intrakardiale Echokardiographie (ICE) festgestellt. Die digitalen Aufnahmen können aus diesen bildgebenden Verfahren unter Verwendung von handelsüblicher CAD-und/oder Bildgebungssoftware rekonstruiert werden. Dabei wird ein dreidimensionaler Bilddatensatz rekonstruiert, der die Öffnung im Herzen, beispielsweise den Septum-Defekt maßstabsgetreu darstellt. Vorteilhafterweise wird die geometrische Information über das Herz in einem dem DICOM Standard (Digital Imaging and Communication in Medicine) entsprechenden Format gespeichert. Aus dem 3D-DICOM Datensatz wird nach Datensegmentierung ein 3D-Objekt gespeichert, welches in ein Format umgewandelt wird, das sich zur Herstellung eines 3D-Modells eignet. Vorzugsweise wird ein STL Format verwendet. Aus dem STL Format wird ein Modell des Septum-Defekts gewonnen, indem ausgehend vom Computer generiertem Modell ein entsprechendes reales Modell gefertigt wird. Die Daten werden beispielsweise an einen 3-D Drucker übermittelt und das Modell des Septum-Defekts wird durch selektives Laser-Sintern hergestellt. Das so hergestellte Modell dient zur Formgebung des Geflechts der Verschlussscheiben sowie zur Herstellung des erfindungsgemäßen Okkluders.

Das Modell kann aus allen temperaturbeständigen Werkstoffen bestehen, die zum Laser-Sintern geeignet sind. In einer Ausführungsform ist das Modell aus Stahl.

Der Ausdruck "die Außenkontur und Größe mindestens einer der der Verschlussscheiben der Außenkontur und Größe des Septum-Defekts entspricht" bedeutet sowohl eine Berücksichtigung der Form und Größe des Septum-Defekts als auch deren Lage in der Vorhof-Scheidewand. Die Verschlussscheibe ist entsprechend dem Septum-Defektrand geformt, ragt jedoch über den Defektrand heraus, was für einen festen Sitz des Okkluders erforderlich ist.

Die erste Verschlussscheibe ist in Gebrauchsstellung linksatrial positioniert. Die zweite Verschlussscheibe ist in Gebrauchsstellung rechtsatrial positioniert. Die linksatriale und die rechtsatriale Verschlussscheibe können gleich geformt sein oder unterschiedliche Formen haben. Vorzugsweise ist die rechtsatrial positionierte Verschlussscheibe etwas grösser geformt, was den Sitz des Okkluders verbessert. Der Ausdruck "etwas größer geformt" bedeutet, dass der Umfang der Verschlussscheibe circa 2-10% größer ist als Umfang des Septum-Defekts.

Die Verschlussscheibe besteht aus einem Geflecht aus Formgedächtnismaterial. Der Ausdruck "Geflecht aus Formgedächtnismaterial" umfasst alle Materialien die nach einer starken Verformung ihre frühere Formgebung wiedererlangen können wie Formgedächtnislegierungen (z. B. Nitinol) und Formgedächtnispolymere (biokompatible Kunststoffgeflechte, absorbierbare Kunststoffe).

In einer Ausführungsform sind die erste und die zweite Verschlussscheibe ein Metallgeflecht aus Nitinol. Das Metallgeflecht ist somit selbstexpandierend und besitzt eine erste zusammengefaltete Konfiguration und eine zweite expandierte Konfiguration.

Die Verschlussscheiben sind vorzugsweise innen mit Kunststoff beschichtet. Dazu eignet sich beispielsweise. Polytetrafluorethylen (PTFE), Dacron, Polyester). Beide Verschlussscheiben können beschichtet sein oder nur eine der Verschlussscheiben.

Das Metallgeflecht besteht beispielsweise aus mindestens 30 Nitinolfäden, vorzugsweise 30-100 Fäden mit einem Durchmesser von 0,03-0,15mmm.

Da der Okkluder auf der Basis eines patientenspezifischen Modells des Septum-Defekts erstellt wird sind die Verschlussscheiben nur in den seltensten Fällen kreisförmig. Die Verschlussscheiben haben die unterschiedlichsten Formen. Die Formen spiegeln die Vielfalt der Anatomie des Herzens wieder. Das Drahtgeflecht aus Formgedächtnismaterial wird vor der zur Formgebung erforderlichen Wärmebehandlung in das Septum-Defektmodell derart eingepasst, dass die Außenkontur und Größe der Verschlussscheiben analog der Außenkontur und Größe des Septum-Defekts ist. Um einen festen Sitz zu gewährleisten müssen die Verschlussscheiben jedoch die Ränder des Septum-Defekts übergreifen, damit die Verschlussscheiben an der Vorhofscheidewand anliegen.

Zur Herstellung des Okkluders wird zunächst ein schlauchförmiges Netze geflochten. Aus dem Netz werden entweder zwei Verschlussscheiben geformt und das Geflechtsende wird in einer Klemme gefasst oder es werden zwei schlauchförmige Netze geflochten aus denen je eine Verschlussscheibe geformt wird deren Enden vorzugsweise miteinander verschweißt werden wie in EP2531113B1 (Osypka) beschrieben. Es ist aber auch möglich die Enden der schlauchförmigen Netze zu fassen und die beiden Fassungen miteinander zu verbinden. Ein derartiger Okkluder wird beispielsweise in EP2116190A1 (Osypka) beschrieben.

Der Kopplungsteil des Okkluders kann geflochten, gekoppelt oder geschweißt sein. Alle im Stand der Technik bekannten Kopplungsmöglichkeiten können eingesetzt werden.

In einer Ausführungsform erfolgt die Verbindung der beiden gefassten Verschlussscheiben über ein elastisches Element, welches als Feder dient wie beispielsweise eine Wendel oder ein Si-Schlauch. Ein solches Federelement erlaubt eine feste Anlage der beiden Scheiben beidseits der Vorhofscheidewand. Unterschiedlich dicke Vorhofscheidewände können so berücksichtigt werden.

Die Verbindung der beiden Verschlussscheiben kann durch zusätzliche Verbindungselemente verstärkt werden. Beispielsweise Magneten oder rasterverbindungen.

Der Okkluder kann ein zentrales Lumen aufweisen. Dazu hat das Drahtgeflecht im Zentrum eine Öffnung. Die Öffnung ist vorzugsweise mit einem selbstdichtenden Ventil versehen, was den Blutfluss vom linken in den rechten Vorhof verhindert, andererseits aber den Durchgang eines Katheters oder einer Elektrode, beispielsweise zur Stimulation des linken Vorhofs ermöglicht. Eine solche Anordnung wird in EP2674189 beschrieben. Selbstdichtende Ventile sind z. B. Druckventile wie Silikonventile, die im Handel in vielen Formen und Größen erhältlich sind.

Die folgenden Figuren erläutern den erfindungsgemäßen Okkluder.

Bezugszeichen:
- 1: Verschlussscheibe linksatrial positionierbar
- 2: Verschlussscheibe rechtsatrial positionierbar
- 3: Kopplungsteil
- 4: Magnet
- 5: Magnet
- 6: Magnet
- 7: Magnet
- 8: Verschlussscheibe Stand der Technik

- 10: Septum-Defekt
- 20: Vorhofscheidewand
- 30: Blutgefäß

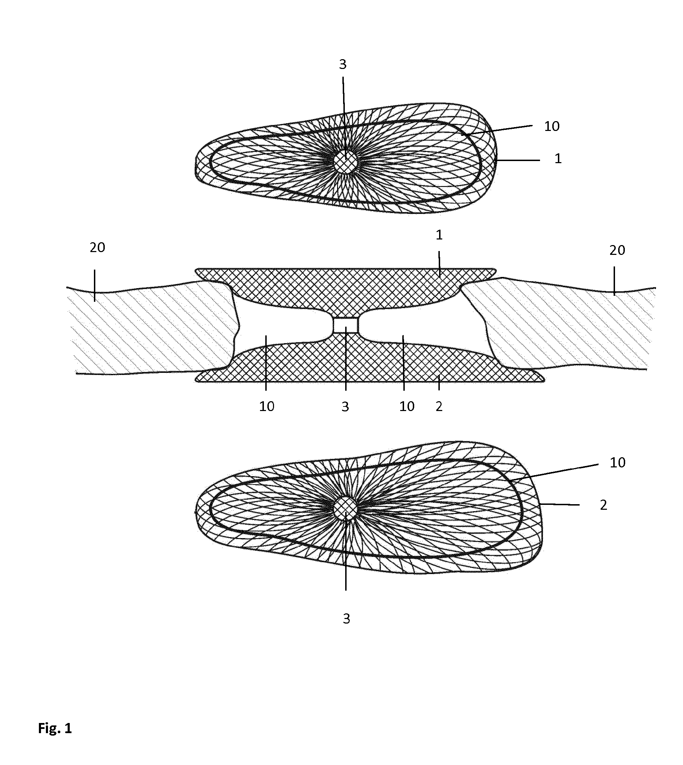

**Fig.1** zeigt in der Aufsicht und im Querschnitt den erfindungsgemäßen Okkluder bestehend aus den Verschlussscheiben (1) und (2), die als Geflecht ausgebildet sind und über das Kopplungsteil (3) miteinander verbunden sind. Verschlussscheibe (2) ist aufgrund der anatomischen Gegebenheiten in der Vorhofscheidewand (20) etwas größer geformt als Verschlussscheibe (1). Beide Verschlussscheiben (1) und (2) entsprechen in ihrer Außenkontur dem Septum-Defekt (10).
   In Gebrauchsstellung sind die Verschlussscheiben (1) und (2) linksatrial und rechtsatrial positionierbar. Das Kopplungsteil (3) durchzieht die Öffnung im Herzen. Die Verschlussscheiben übergreifen die Ränder der ungewollten Öffnung im Herzen.

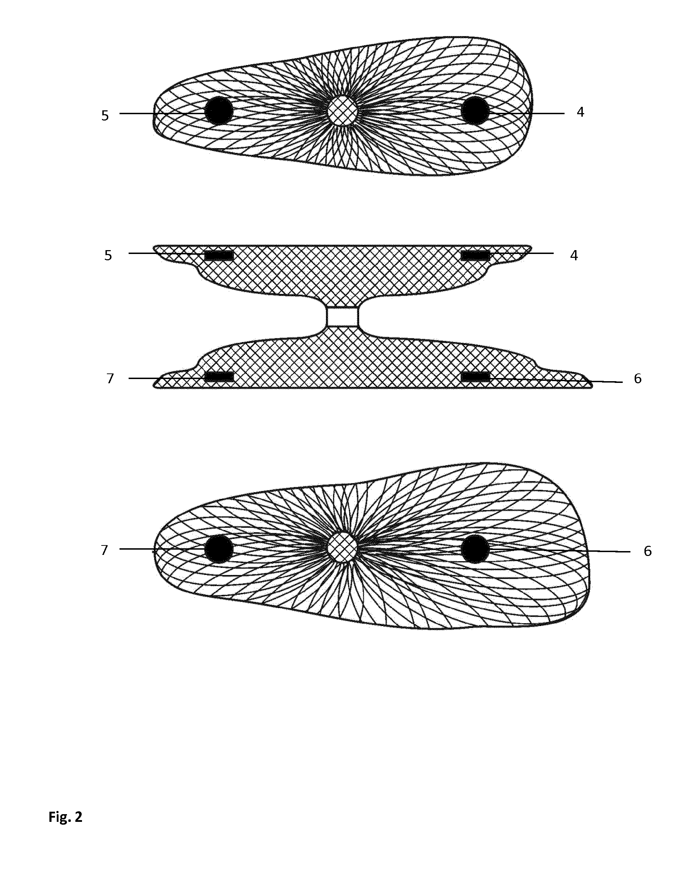

**Fig. 2** zeigt den in Fig. 1 dargestellten Okkluder. Um den Halt der Verschlussscheiben zu verbessern sind im Randbereich der Verschlussscheibe je zwei Magnete (4) und (5) bzw. (6) und (7) angebracht.
   Die Magnete (4) und (5) liegen auf der Längsachse von Verschlussscheibe (1). Die Magnete (6) und (7) liegen auf der Längsachse von Verschlussscheibe (2). Magnet (4) liegt in Gebrauchsstellung direkt gegenüber von Magnet (6). Entsprechend liegt Magnet (5) gegenüber von Magnet (7)

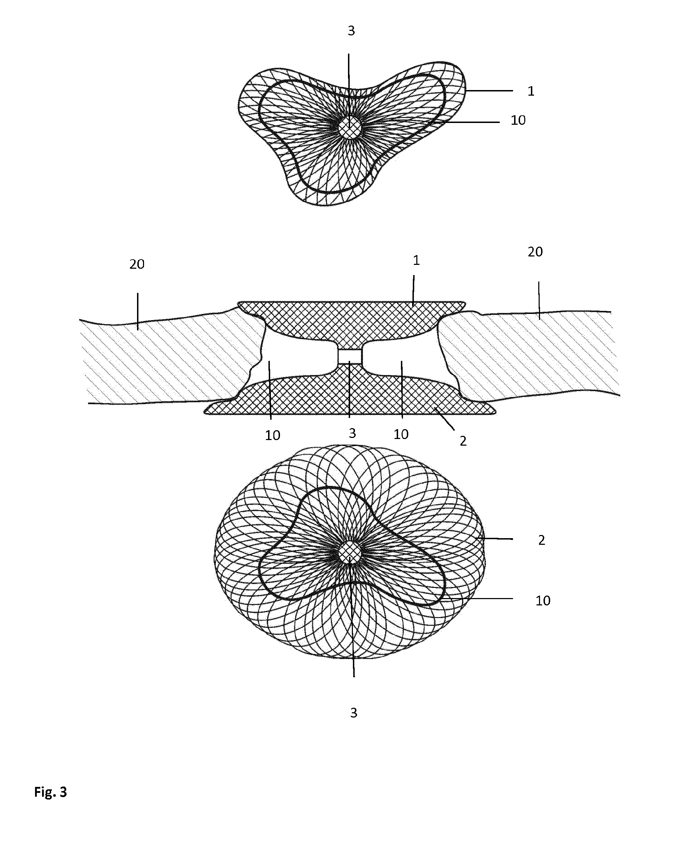

**Fig. 3** zeigt ebenso wie Fig. 1 die linksatrial positionierbare Verschlussscheibe (1) und die rechtsatrial positionierbare Verschlussscheibe (2) in der Aufsicht und im Querschnitt. Die Bezugszeichen entsprechen den in Fig. 1 verwendeten Bezugszeichen. In dieser Ausführungsform hat die rechtsatrial positionierbare Verschlussscheibe (2) eine andere Form als die linksatrial positionierbare Verschlussscheibe (1). Verschlussscheibe (2) ist rund und entspricht somit gebräuchlichen runden Verschlussscheiben. Verschlussscheibe (1) entspricht in ihrer Außenkontur dem Septum-Defekt (10). Eine solche Kombination ist dann möglich, wenn die anatomischen Gegebenheiten eine größere rechtsatrial positionierbare Verschlussscheiben (2) zulassen, das heißt wenn im rechten Vorhof kein Blutgefäß durch die Verschlussscheibe tangiert wird.
**Fig. 4** zeigt einen Schnitt durch ein 3 D Modell des Herzens im Bereich der Vorhöfe. Das 3-D Modell stellt ein Patienten spezifisch erstelltes Modell des Septum-Defekts dar, welches aufgrund von Patientendaten aufgenommen wurde. Man erkennt dass das Loch (10) in der Vorhofscheidewand relativ groß ist und nahe an ein großes Blutgefäß (30) (z.B. Pulmonalaterie) heranreicht. Man erkennt ferner die Verschlussscheibe (1) des Okkluders, die linksatrial positioniert ist. Die Verschlussscheibe (1) entspricht in Form und Größe dem Septum-Defekt. Die Außenkontur der Verschlussscheibe (1) folgt der Außenkontur des Loches (10). Die Ränder der Verschlussscheibe (1) ragen über den Defektrand heraus, was für einen festen Sitz des Okkluders erforderlich ist. Die Verschlussscheibe sieht man in der Aufsicht, sodass der Kopplungsteil (3) zur rechtsatrial positionierten Verschlussscheibe als kleiner zentral gelegener Kreis erscheint.

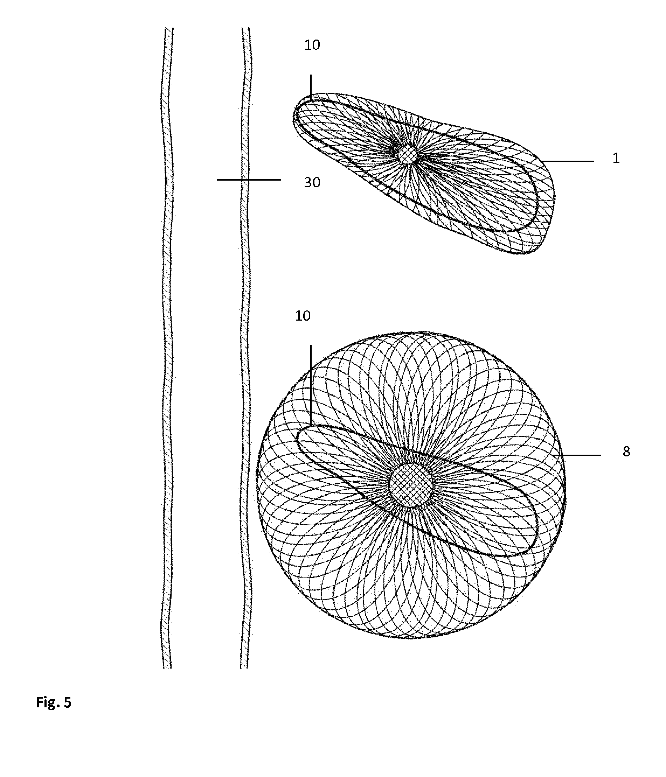

**Fig. 5** zeigt schematisch den in Fig. 1 im Modell dargestellten Septum-Defekt. Der Septum-Defekt (10) verläuft nahe an einem Blutgefäß (30). Verschlussscheibe (1) entspricht in ihrer Außenkontur dem Septum-Defekt wie in Fig. 4 beschrieben. Verschlussscheibe (8) ist ein derzeit gebräuchlicher kreisrunder Okkluder, wie er im Stand der Technik beschrieben wird. Man erkennt klar, dass zur Abdeckung des ovalen Septum-Defekts die kreisrunde Verschlussscheibe einen relativ großen Durchmesser haben muss. Der Durchmesser des Okkluders muss etwas grösser sein als die Längsachse des ovalen Defekts.
   Diese schematische Zeichnung erklärt das oben beschriebene Problem von übergroßen Okkludern. Der Rand der Verschlussscheibe (8) tangiert quasi die Wand des Blutgefäßes (30), sodass das Geflecht bei der Bewegung des schlagenden Herzens an der Wand des Blutgefäßes reibt, was zu der gefürchteten Erosion führt.

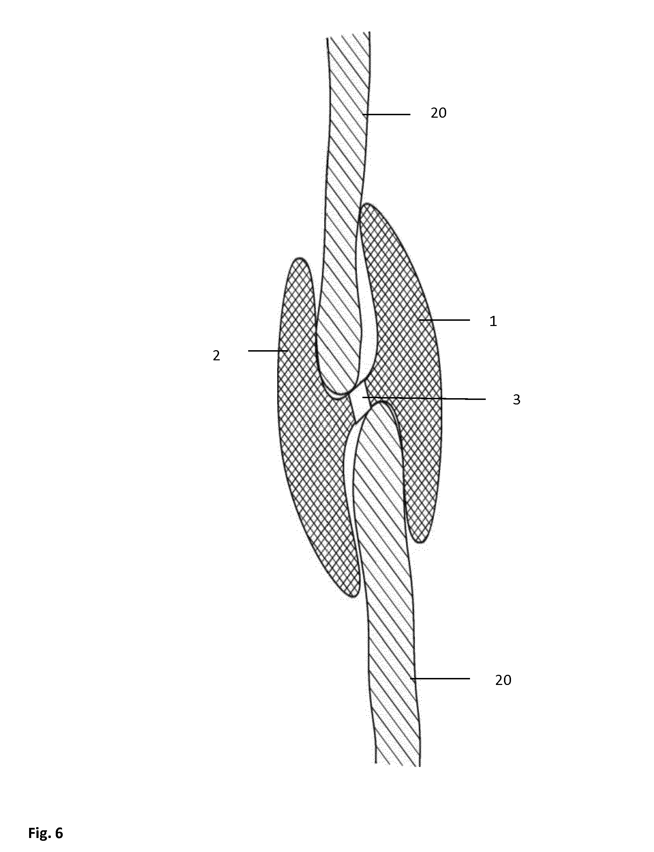

**Fig. 6** zeigt einen erfindungsgemäßen Okkluder, der sich zum Verschließen eines PFO besonders gut eignet. Verschlussscheibe (1) und Verschlussscheibe (2) sind über das Kopplungsteil (3) derart miteinander verbunden, dass die Verschlussscheiben zentrisch versetzt sind. Dies entspricht der Anatomie des PFO. Der Defekt im Foramen ovale ist ein "Loch" in der Vorhofscheidewand (20), das überlappende feine Septen gekennzeichnet ist.

## Patentansprüche

1. Patienten spezifisch geformter Okkluder geeignet zum Verschließen einer ungewollten Öffnung im Herzen wobei der Okkluder von einer kompakten Erscheinungsform in eine expandierte Erscheinungsform überführbar ist, wobei der Okkluder eine erste Verschlussscheibe (1) und eine zweite Verschlussscheibe (2) aufweist, welche in der expandierten Erscheinungsform auf je einer Seite der Öffnung anliegen, und wobei der Okkluder ferner einen Kopplungsteil (3) aufweist welcher die beiden Verschlussscheiben (1) und (2) verbindet, **dadurch gekennzeichnet dass** die Außenkontur und Größe mindestens einer der Verschlussscheiben der Außenkontur und Größe des Septum-Defekts (10) entspricht, wobei ein Patienten spezifisch erstelltes Modell des Septum-Defekts zur Herstellung und Formgebung der Verschlussscheiben dient.

2. Okkluder gemäß Anspruch 1, wobei die Verschlussscheiben (1) und (2) aus einem Geflecht aus Formgedächtnismaterial bestehen.

3. Okkluder gemäß Anspruch 2, wobei die Verschlussscheiben (1) und (2) aus Nitinol bestehen.

4. Okkluder gemäß Anspruch 2 oder 3, wobei das Geflecht mindestens einer der Verschlussscheiben (1) oder (2) mit einer Kunststoffmembran überzogen ist.

5. Okkluder gemäß einem der Ansprüche 1-4, wobei die Außenkontur und Größe beider Verschlussscheiben (1) und (2) der Außenkontur und Größe des Septum-Defekts (10) entspricht.

6. Okkluder gemäß einem der Ansprüche 1-4, wobei die Außenkontur einer der Verschlussscheiben circa 2-10% größer ist.

7. Okkluder gemäß einem der Ansprüche 1-6, wobei die Verschlussscheiben (1) und (2) miteinander verschweißt sind.

8. Okkluder gemäß Anspruch 1, wobei im Randbereich jeder Verschlussscheibe je zwei Magneten angebracht sind.

9. Okkluder gemäß Anspruch 1, wobei der Okkluder ein zentrales Lumen und ein selbstdichtendes Ventil aufweist.

10. Verfahren zur Herstellung des Okkluders gemäß Anspruch 1, wobei das Verfahren die folgenden Schritte umfasst
a) Bereitstellung eines Computer generierten 3-D Modells des Septum-Defekts auf der Basis von medizinischen Aufnahmen des Septum-Defekts,
b) Übermittlung der Daten des Computer generierten Modells an einen 3-D Drucker,
c) Herstellung eines realen Modells des Septum-Defekts durch selektives Laser-Sintern;
d) Herstellung eines Geflechts aus Formgedächtnismaterial und Formgebung des Geflechts durch Pressen des Geflechts in das in Schritt c) erhaltene Modell wobei durch das Pressen die Verschlussscheiben entstehen, deren Form der Anatomie des Septum-Defekts maßstabsgetreu entspricht;
e) tempern des Geflechts (1) zur Fixierung der Formänderung,
f) verbinden der Verschlussscheiben.

11. Okkluder gemäß Anspruch 1, hergestellt nach einem Verfahren gemäß Anspruch 10.
